(19) Europäisches Patentamt
European Patent Office
Office européen des brevets

(11) **EP 4 570 897 A1**

(12) **EUROPEAN PATENT APPLICATION**
published in accordance with Art. 153(4) EPC

(43) Date of publication:
**18.06.2025 Bulletin 2025/25**

(21) Application number: **22954916.7**

(22) Date of filing: **09.08.2022**

(51) International Patent Classification (IPC):
***C12M 1/00*** *(2006.01)*

(52) Cooperative Patent Classification (CPC):
**C12M 1/00**

(86) International application number:
**PCT/JP2022/030360**

(87) International publication number:
**WO 2024/033994 (15.02.2024 Gazette 2024/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **HITACHI HIGH-TECH CORPORATION**
**Tokyo 105-6409 (JP)**

(72) Inventors:
• **SATOH Wataru**
**Tokyo 100-8280 (JP)**

• **SATO Koma**
**Tokyo 100-8280 (JP)**
• **SHIBAHARA Masashi**
**Tokyo 105-6409 (JP)**
• **MAKINO Yoko**
**Tokyo 105-6409 (JP)**
• **KUMAZAKI Nobutaka**
**Tokyo 105-6409 (JP)**
• **ISOSHIMA Nobuyuki**
**Tokyo 105-6409 (JP)**

(74) Representative: **Strehl & Partner mbB**
**Maximilianstrasse 54**
**80538 München (DE)**

(54) **TEMPERATURE CONTROL DEVICE AND BIOCHEMICAL DEVICE, ANALYSIS DEVICE, AND GENETIC TESTING DEVICE COMPRISING SAME**

(57) A temperature control device includes a housing, a partition plate that partitions the housing into an upper part and a lower part, a temperature control unit, and an exhaust fan. The temperature control unit includes a temperature adjustment unit and a multi-well plate. The multi-well plate is disposed above the partition plate. The temperature adjustment unit is disposed below the partition plate. An air intake port for introducing outside air is provided on a side surface part of the housing at a predetermined portion located above the partition plate. The partition plate is provided with an exhaust port. The exhaust fan is disposed in such a manner as to discharge air from an exhaust path located below the partition plate to the outside of the housing. The multi-well plate is disposed between the air intake port and the exhaust port. The air flows into the housing from the air intake port, passes above the multi-well plate, and flows into the exhaust path from the exhaust port. Accordingly, variations in the amount of evaporation of a solution held in each of a plurality of wells provided in the multi-well plate can be reduced.

[FIG.1]

EP 4 570 897 A1

## Description

Technical Field

The present disclosure relates to a temperature control device and a biochemical device, an analysis device, and a genetic testing device including the same.

Background Art

In a fully automatic genetic testing device, extraction of a sample containing DNA (deoxyribonucleic acid), mixing of reagents, amplification of DNA, and inspection are fully automatically performed. In this device, high speed of each operation, improvement of analysis accuracy, and the like are required, and rapid progress in performance has been achieved.

The fully automatic genetic testing device has a temperature control device that heats a sample and a reagent to a high temperature to facilitate the reaction between the sample and the reagent.

In the temperature control device, in the case where a solution sample is heated, the solvent evaporates, and the liquid volume changes. When the liquid volume changes, the sample concentration changes, and the analysis accuracy decreases.

Patent Document 1 discloses a biochemical processing device that uses a container having a plurality of container parts capable of containing liquid from the viewpoint of preventing liquid evaporation caused by high temperature heating and contamination of the liquid in a plurality of containers, and that has means for selectively sealing the container parts.

In addition, Patent Document 2 discloses a drying suppressing tool for use in a cell culture kit installed in an incubator in which dust and dirt are suppressed, which has a configuration in which an internal space surrounded by a liquid storage tank and a lid communicates with the outside in a state where the lid is closed, and can suppress water evaporation from a medium held by a well plate.

Prior Art Document

Patent Documents

Patent Document 1: JP-2007-97476-A
Patent Document 2: JP-2022-2505-A

Summary of the Invention

Problems to be Solved by the Invention

The biochemical processing device described in Patent Document 1 has a lid member for the container holding a solution to seal the container, and has a structure for suppressing the amount of evaporation by sealing the container. However, since the lid member and movable means for moving the lid member need to be added, the structure disadvantageously becomes complicated.

In Patent Document 2, adopted is a structure in which liquid is stored around a container containing a sample solution and the outside air communicates through a section containing the liquid. Accordingly, the periphery of the container becomes humidified atmospheric air, and the amount of evaporation from the container is suppressed. However, there is a problem that the liquid needs to be held around a reaction vessel, and such maintenance as addition or replacement of the liquid is required.

An object of the present disclosure is to reduce variations in the amount of evaporation of a solution held in each of a plurality of wells provided in a multi-well plate.

Means for Solving the Problems

A temperature control device of the present disclosure includes a housing, a partition plate that partitions the housing into an upper part and a lower part, a temperature control unit, and an exhaust fan. The temperature control unit includes a temperature adjustment unit and a multi-well plate. The multi-well plate is disposed above the partition plate. The temperature adjustment unit is disposed below the partition plate. An air intake port for introducing outside air is provided on a side surface part of the housing at a predetermined portion located above the partition plate. The partition plate is provided with an exhaust port. The exhaust fan is disposed in such a manner as to discharge air from an exhaust path located below the partition plate to the outside of the housing. The multi-well plate is disposed between the air intake port and the exhaust port. The air flows into the housing from the air intake port, passes above the multi-well plate, and flows into the exhaust path from the exhaust port.

Advantages of the Invention

According to the present disclosure, it is possible to reduce variations in the amount of evaporation of a solution held in each of a plurality of wells provided in a multi-well plate.

Brief Description of the Drawings

FIG. 1 is a schematic longitudinal cross-sectional view for depicting a temperature control device of a first embodiment.
FIG. 2 is a transverse cross-sectional view for depicting the inside of a temperature control device 1 of FIG. 1.
FIG. 3 is a schematic perspective view for depicting a multi-well plate 5 and a temperature adjustment block 6 of FIG. 2.
FIG. 4 is a top view for depicting arrangement of wells 11 in the multi-well plate 5 of FIG. 3.
FIG. 5 is a K-K' cross-sectional view of FIG. 4.
FIG. 6 is a transverse cross-sectional view for depicting the inside of the temperature control device 1 of a second embodiment.
FIG. 7 is a transverse cross-sectional view for depicting the inside of the temperature control device 1 of a third embodiment.
FIG. 8 is a schematic longitudinal cross-sectional view for depicting the temperature control device of a fourth embodiment.
FIG. 9 is a transverse cross-sectional view for depicting the inside of the temperature control device 1 of FIG. 8.
FIG. 10 is a schematic longitudinal cross-sectional view for depicting the temperature control device of a fifth embodiment.
FIG. 11 is a schematic longitudinal cross-sectional view for depicting the temperature control device of a sixth embodiment.
FIG. 12 is a transverse cross-sectional view for depicting the inside of the temperature control device 1 of FIG. 11.

Modes for Carrying Out the Invention

Hereinafter, embodiments will be described by using the drawings.

[First Embodiment]

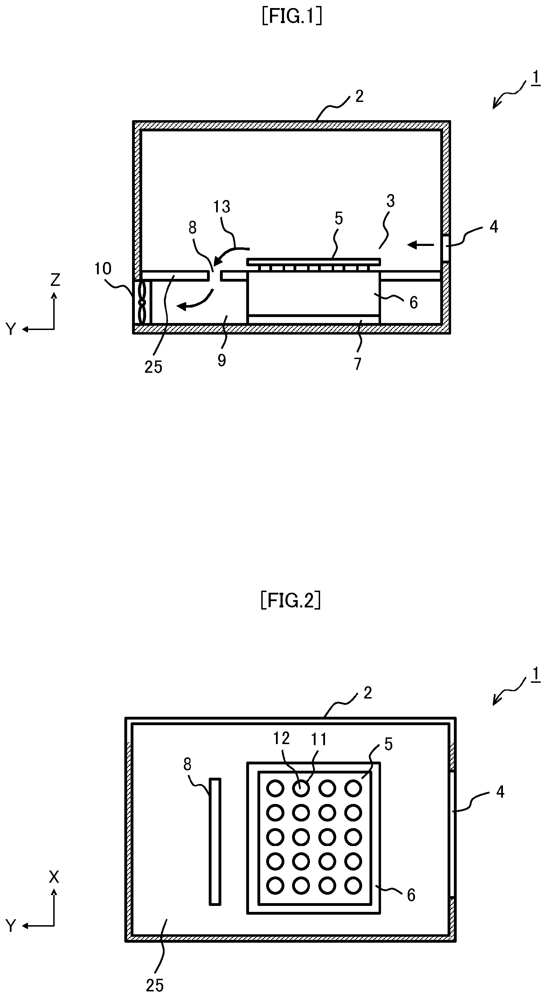

FIG. 1 is a schematic longitudinal cross-sectional view for depicting a temperature control device of a first embodiment.
In the drawing, a temperature control device 1 is configured with a housing 2, a temperature control unit 3, a partition plate 25 that partitions the housing 2 into an upper part and a lower part, and an exhaust fan 10. An air intake port 4 for introducing outside air is provided on a side surface part of the housing 2 at a predetermined portion located above the partition plate 25. The partition plate 25 is provided with an exhaust port 8. The exhaust fan 10 is disposed in such a manner as to discharge air from an exhaust path 9 provided below the partition plate 25.
The temperature control unit 3 includes a temperature adjustment unit 7, a temperature adjustment block 6, and a multi-well plate 5. The multi-well plate 5 is disposed above the partition plate 25. The temperature adjustment unit 7 and the temperature adjustment block 6 are disposed below the partition plate 25. In other words, the temperature control unit 3 is inserted into the partition plate 25. It should be noted that, in this specification, "temperature adjustment" is an abbreviation of "adjustment of temperature."
With the configuration described above, the outside air introduced from the air intake port 4 passes above the partition plate 25 and the multi-well plate 5 as an airflow 13, passes through the exhaust port 8 and the exhaust path 9, and is discharged to the outside from the exhaust fan 10.
FIG. 2 is a transverse cross-sectional view for depicting the inside of the temperature control device 1 of FIG. 1.
As depicted in FIG. 2, the multi-well plate 5 has a plurality of wells 11. Solutions 12 are dispensed into the respective wells 11. The respective solutions 12 are so held as not to mix with each other.

In the drawing, the exhaust port 8 has an elongated rectangular shape.

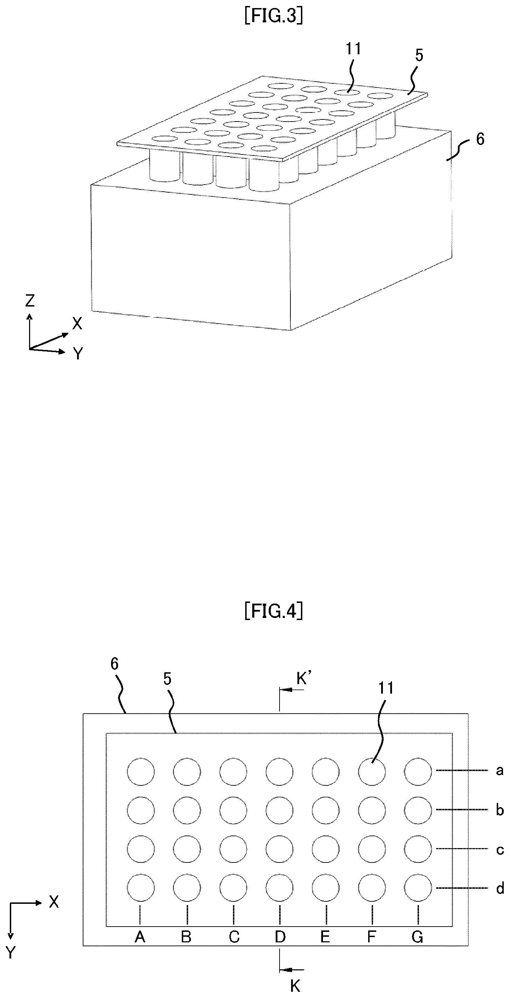

FIG. 3 is a schematic perspective view for depicting the multi-well plate 5 and the temperature adjustment block 6 of FIG. 2.

As depicted in FIG. 3, the multi-well plate 5 is supported by the temperature adjustment block 6. Each well 11 is inserted into the temperature adjustment block 6.

FIG. 4 is a top view for depicting arrangement of the wells 11 in the multi-well plate 5 of FIG. 3.

In FIG. 4, regarding the arrangement of the wells 11 formed in the multi-well plate 5, columns are defined in a positive direction of an X-axis and denoted by A, B, C, D, E, F, and G, and rows are defined in a positive direction of a Y-axis and denoted by a, b, c, and d. For example, the well 11 in the column A and the row a is expressed as "Aa," and the well 11 in the column G and the row c is expressed as "Gc."

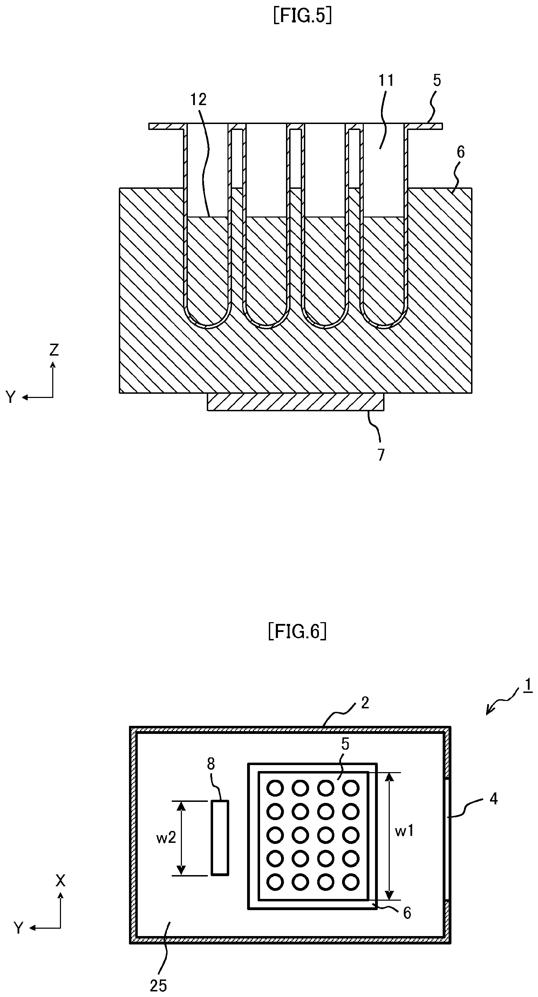

FIG. 5 is a K-K' cross-sectional view of FIG. 4.

As depicted in FIG. 5, the wells 11 used as test tubes are formed in the multi-well plate 5. The solutions 12 are injected into the respective wells 11.

Here, the amount of evaporation of the solution 12 can be obtained by the following calculation procedure.

The amount of evaporation $Q_{evap}$ [kg/m$^3$/s] from the surface of the solution 12 to the surrounding air is obtained by the following equation (1).

[Math. 1]

$$Q_{evap} = k(Y_w - Y_a) \quad \ldots \text{ Equation (1)}$$

Here, k [m/s] is a mass transfer coefficient, $Y_w$ [kg/m$^3$] is the amount of saturated steam at the water surface at a liquid temperature $T_w$ [°C], and $Y_a$ [kg/m$^3$] is the amount of water vapor of the surrounding air at an air temperature $T_a$ [°C] and at humidity HR [%].

$Y_w$ can be obtained by using the following equation (2).

[Math. 2]

$$Y(T_a) = 217 \cdot \frac{e(T_a)}{T_a + 273.15} \quad \ldots \text{ Equation (2)}$$

Here, $e(T_a)$ is a saturated water vapor pressure and can be obtained by using the Wagner's equation of saturated water vapor pressure indicated in the following equation (3).

[Math. 3]

$$e(T_a) = 221200 \cdot exp\left(\frac{-7.76451x + 1.45838x^{1.5} - 2.7758x^3 - 1.23303Dx^6}{1-x}\right).$$

$$\ldots \text{ Equation (3)}$$

Here, D [m$^2$/s] is a diffusion coefficient and can be calculated by using a relational expression of Sherwood number Sh that is a dimensionless quantity related to mass transfer expressed by the following equation (4).

[Math. 4]

$$Sh = \frac{kL}{D} = 0.664 Sc^{1/3} Re^{1/2}$$

$$\ldots \text{ Equation (4)}$$

Here, Sc is a Schmidt number and is the ratio of kinematic viscosity and the diffusion coefficient of liquid obtained by the following equation (5).

[Math. 5]

$$Sc = \frac{\mu}{\rho D}$$

... Equation (5)

Note that $\mu$ [Pa·s] is a viscosity coefficient, and $\rho$ [kg/m³] is density. In addition, a Reynolds number Re used in the above equation (4) is the ratio of an inertial force and a viscous force obtained in the following equation (6).
[Math. 6]

$$Re = \frac{\rho u L}{\mu}$$

... Equation (6)

Note that L [m] is a representative length, and u [m/s] is a wind speed.

From these equations, an influence of an air flow speed on a water evaporation speed can be quantitatively calculated. That is, when air having no unevenness in temperature and humidity flows above the wells 11 formed in the multi-well plate 5 at a uniform flow speed, the amounts of evaporation $Q_{evap}$ in the respective wells 11 are equal.

In addition, the amount of evaporation from the surface of the solution 12 to the surrounding air increases as the temperature of the flowing air is higher and the flow speed is faster. Meanwhile, the amount of evaporation increases as the humidity of the flowing air is lower.

In general, when the temperature control unit 3 is heated to be higher in temperature than the surrounding air, an updraft is generated, and natural convection is generated therearound. In the structure of the present embodiment, the air flows in a positive direction of a Z-axis in a central part of the multi-well plate 5. Then, the air flows from an outer peripheral part of the multi-well plate 5 toward the central part.

Accordingly, the air around the temperature control unit 3 passes above the wells 11 disposed at the outer peripheral part of the multi-well plate 5, and thus the evaporation of the solution 12 is accelerated. This increases the humidity of the air that has passed above the wells 11. The air with increased humidity flows to the central part of the multi-well plate 5. Since the air flow speed is small above the wells 11 in the central part, the amount of evaporation in the central part is smaller than that in the outer peripheral part.

Described with reference to FIG. 4, in the case where no countermeasure is taken, the amount of evaporation in the column A is larger than that in the column C due to natural convection. In addition, the amounts of evaporation in the row a and the row d are larger than those in the row b and the row c. Further, since unhumidified air flows into corner parts of the multi-well plate 5 from both directions of the X-axis direction and the Y-axis direction, the amounts of evaporation are the largest in the corner parts. That is, the amounts of evaporation in the wells Aa, Ad, Ga, and Gd increase, and variations in the amounts of evaporation in the respective wells 11 increase. The amounts of evaporation vary even in the same column A or G.

Meanwhile, in the configuration depicted in FIG. 1 and FIG. 2, outside air flows in from the air intake port 4 formed in the housing 2, and the airflow 13 is generated above the multi-well plate 5. That is, the airflow 13 is generated above the solutions 12 held in the multi-well plate 5 in the positive direction of the Y-axis (left direction in the drawings).

As a result, as depicted in FIG. 4, the amount of evaporation of the solution 12 is the largest in the row a, the amount of evaporation gradually decreases, and the amount of evaporation is the smallest in the row d. In this case, since no flow in the X-axis direction is generated above the multi-well plate 5, the amounts of evaporation of the solutions 12 from the column A to the column G can be made substantially uniform in each of the row a to the row d.

By generating the uniform airflow above the container in this manner, it is possible to suppress variations in the amounts of evaporation among the columns. That is, it is possible to suppress variations in the amounts of evaporation in the same row, for example, from Aa to Ga.

Here, the air intake port 4 and the exhaust port 8 may each be provided separately at two or more places. In addition, the multi-well plate 5 is not limited to the structure in which the plurality of wells 11 are integrated, and may be a structure in which a plurality of independent wells 11 are fixed to the temperature adjustment block 6. In addition, the exhaust air volume by the exhaust fan 10 need not be constant, and the air volume may be changed according to the environmental temperature and environmental humidity. The temperature adjustment block 6 is desirably formed of a material having high thermal conductivity, and, for example, an aluminum alloy, a copper alloy, a magnesium alloy, or the like is preferably used.

The temperature adjustment unit 7 may be configured with a heater, a Peltier element, or a heat pump.

[Second Embodiment]

In the present embodiment, the width of the exhaust port 8 is made narrower than that of the multi-well plate 5. It should be noted that the descriptions of the configurations denoted by the same symbols and the parts having the same functions described in the first embodiment are omitted.

FIG. 6 is a transverse cross-sectional view for depicting the inside of the temperature control device 1 of a second embodiment.

In the drawing, a width w2 of the exhaust port 8 in the X-axis direction and a width w1 of the multi-well plate 5 in the X-axis direction are set to satisfy w1 > w2.

By narrowing the width w2 of the exhaust port 8 in this manner, the range in which the flow speed of the airflow 13 (see FIG. 1) flowing above the multi-well plate 5 is high is limited to central columns of the multi-well plate 5, and the flow speed above the column B to the column F is faster than that near the column A and the column G. As a result, the amounts of evaporation in the column B to the column F increase, the difference from the amounts of evaporation in the column A and the column G where the amounts of evaporation increase not only due to the flow from the air intake port 4 but also due to the influence of natural convection is reduced, and variations in the amounts of evaporation in the respective wells 11 of the multi-well plate 5 can be suppressed.

More specifically, as depicted in the drawing, it is desirable that the width w2 of the exhaust port 8 be made narrower, for example, than the arrangement of the outermost wells 11 in the multi-well plate 5. That is, by making w2 equal to the width ranging from the column B to the column F, it is possible to make the amounts of evaporation in the outermost column A and column G substantially equal to those in the column B to the column F.

[Third Embodiment]

In the present embodiment, the shape of the exhaust port 8 is changed to a trapezoidal shape. It should be noted that the descriptions of the configurations denoted by the same symbols and the parts having the same functions described in the first and second embodiments are omitted.

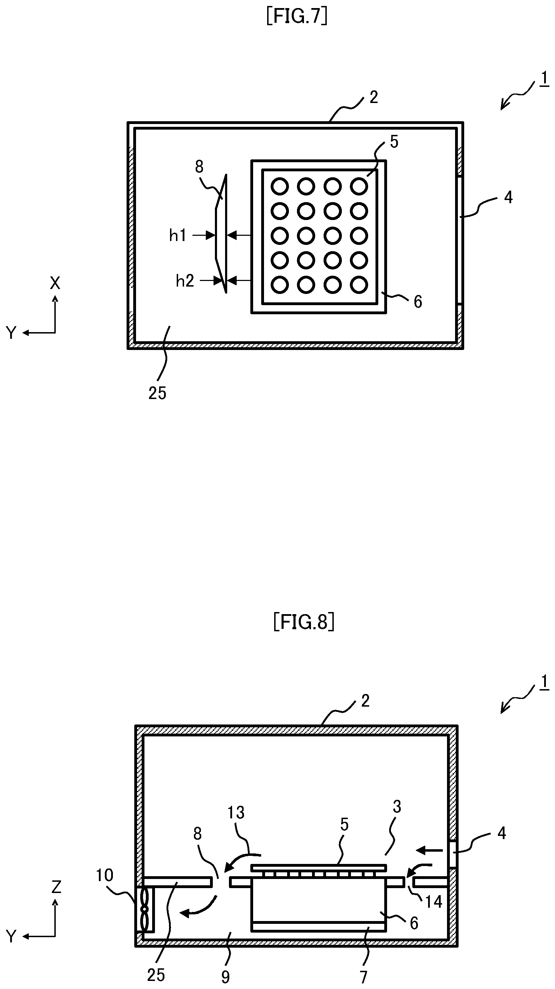

FIG. 7 is a transverse cross-sectional view for depicting the inside of the temperature control device 1 of a third embodiment.

In the drawing, the exhaust port 8 has a trapezoidal shape in which the width in the X-axis direction becomes smaller toward the positive direction of the Y-axis (the advancing direction of the airflow 13 (see FIG. 1)). A depth h1 in the Y-axis direction in a central part in the X-axis direction and a depth h2 at each end in the X-axis direction are set to satisfy h1 > h2.

By narrowing the depth h2 of the exhaust port 8 in this manner, distribution is generated in the flow speeds in the exhaust port 8, and the flow speed is large in the central part of the exhaust port 8 and small at each end of the exhaust port 8. Accordingly, the airflow 13 flowing above the multi-well plate 5 is larger in central columns of the multi-well plate 5, and the flow speed above the column B to the column F is faster than that near the column A and the column G. As a result, the amounts of evaporation in the column B to the column F increase, the difference from the amounts of evaporation in the column A and the column G where the amounts of evaporation increase not only due to the flow from the air intake port 4 but also due to the influence of natural convection is reduced, and variations in the amounts of evaporation in the respective wells 11 of the multi-well plate 5 can be suppressed.

Here, the shape of the opening is not limited to the shape that linearly narrows from h1 to h2, and may be a shape in which three or more depths are combined, a shape that narrows in an arc shape, a shape that narrows stepwise, or other shapes.

[Fourth Embodiment]

In the present embodiment, two exhaust ports are disposed on the upstream side and the downstream side of the multi-well plate 5. It should be noted that the descriptions of the configurations denoted by the same symbols and the parts having the same functions described in the first to third embodiments are omitted.

FIG. 8 is a schematic longitudinal cross-sectional view for depicting the temperature control device of a fourth embodiment.

In the drawing, unlike FIG. 1, a front exhaust port 14 is provided between the air intake port 4 and the temperature control unit 3. In addition, the temperature adjustment unit 7 is not in contact with the bottom of the housing 2. In other words, a gap is provided below the temperature adjustment unit 7. The air sucked from the front exhaust port 14 passes through the exhaust path 9 and is discharged to the outside of the housing 2 by the exhaust fan 10. Other configurations are the same as those of FIG. 1.

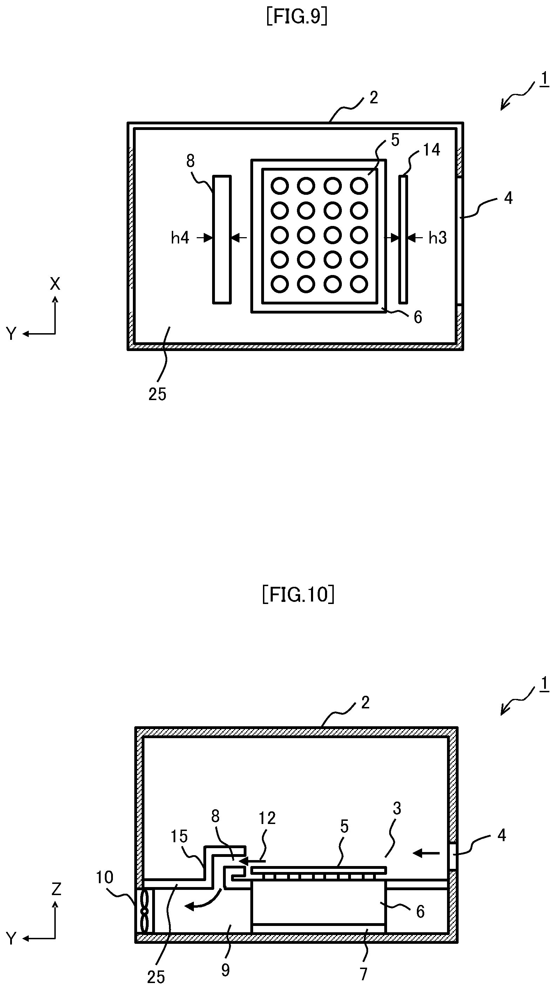

FIG. 9 is a transverse cross-sectional view for depicting the inside of the temperature control device 1 of FIG. 8.

In FIG. 9, a depth h3 of the front exhaust port 14 in the Y-axis direction and a depth h4 of the exhaust port 8 in the Y-axis direction are set to satisfy h3 < h4.

By narrowing the depth of the front exhaust port 14 in this manner, pressure loss in the front exhaust port 14 increases, and the flow speed decreases.

In contrast, since the depth h4 of the exhaust port 8 is large, the air volume is large, and the flow speed is large. The amount of air sucked from the exhaust port 8 is larger than that exhausted from the front exhaust port 14.

Therefore, the airflow 13 flowing in from the air intake port 4 passes above the multi-well plate 5, and variations in the amounts of evaporation of the solutions in the multi-well plate 5 can be suppressed.

In addition, according to the present embodiment, since the amount of the airflow 13 passing above the multi-well plate 5 can be limited, it is possible to prevent droplets of the solution from diffusing.

[Fifth Embodiment]

In the present embodiment, a duct is provided for the exhaust port. It should be noted that the descriptions of the configurations denoted by the same symbols and the parts having the same functions described in the first to fourth embodiments are omitted.

Described here is an example of a structure formed up to substantially the same height as the multi-well plate 5. Descriptions will be omitted for the already described configurations denoted by the same symbols and the parts having the same functions.

FIG. 10 is a schematic longitudinal cross-sectional view for depicting the temperature control device of a fifth embodiment.

In the drawing, a duct part 15 is provided for the exhaust port 8, and the opening of the exhaust port 8 is set at a height substantially equal to an upper surface of the multi-well plate 5.

With such a configuration, the step between the exhaust port 8 and the upper surface of the multi-well plate 5 can be eliminated, the airflow 13 introduced from the air intake port 4 and passing above the multi-well plate 5 can be sucked into the duct part 15 without curving, the uniformity of the flow above the multi-well plate 5 can be improved, and variations in the amounts of evaporation of the solutions can be suppressed.

The shape of the duct part 15 is not limited to be substantially the same shape as the exhaust port 8 and may be a circular tube shape or a duct shape narrower than the exhaust port 8.

[Sixth Embodiment]

In the present embodiment, a movable lid part is provided such that the width of the exhaust port can be changed. It should be noted that the descriptions of the configurations denoted by the same symbols and the parts having the same functions described in the first to fifth embodiments are omitted.

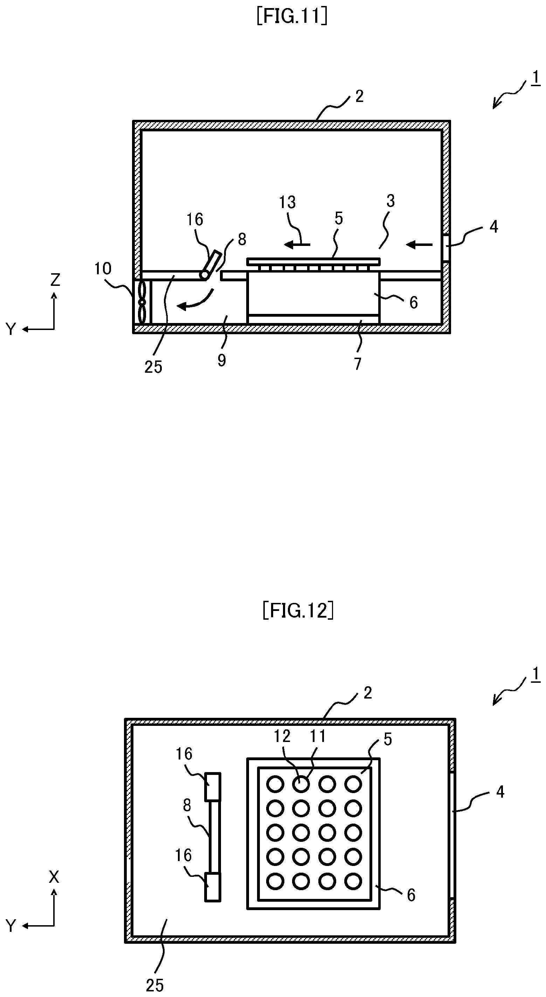

FIG. 11 is a schematic longitudinal cross-sectional view for depicting the temperature control device of a sixth embodiment.

In the drawing, the exhaust port 8 is provided with a movable lid part 16.

FIG. 12 is a transverse cross-sectional view for depicting the inside of the temperature control device 1 of FIG. 11.

As depicted in FIG. 12, the movable lid part 16 is provided at each end of the exhaust port 8 in the width direction (X-axis direction). The lid part 16 can change the width of the exhaust port 8 by the opening and closing thereof being controlled. In addition, by controlling the angle of the lid part 16, not only simple opening and closing but also fine adjustment of flow path resistance can be made.

According to the embodiments described above, it is possible to adjust the flow speed distribution of the airflow 13 above the multi-well plate 5 by adjusting the flow speed in the central part and the flow speed at each end in the exhaust port 8 according to the ambient humidity and the outside air temperature. Accordingly, variations in the amounts of evaporation of the solutions 12 held in the multi-well plate 5 can be suppressed.

It should be noted that the temperature control device according to the present disclosure is applicable to a biochemical device, an analysis device, and a genetic testing device.

Description of Reference Symbols

1:     Temperature control device
2:     Housing
3:     Temperature control unit
4:     Air intake port
5:     Multi-well plate
6:     Temperature adjustment block
7:     Temperature adjustment unit
8:     Exhaust port

**EP 4 570 897 A1**

9: Exhaust path
10: Exhaust fan
11: Well
12: Solution
13: Airflow
14: Front exhaust port
15: Duct part
16: Lid part
25: Partition plate

**Claims**

1.  A temperature control device comprising:

    a housing;
    a partition plate that partitions the housing into an upper part and a lower part;
    a temperature control unit; and
    an exhaust fan,
    wherein the temperature control unit includes a temperature adjustment unit and a multi-well plate,
    the multi-well plate is disposed above the partition plate,
    the temperature adjustment unit is disposed below the partition plate,
    an air intake port for introducing outside air is provided on a side surface part of the housing at a predetermined portion located above the partition plate,
    the partition plate is provided with an exhaust port,
    the exhaust fan is disposed in such a manner as to discharge air from an exhaust path located below the partition plate to an outside of the housing,
    the multi-well plate is disposed between the air intake port and the exhaust port, and
    the air flows into the housing from the air intake port, passes above the multi-well plate, and flows into the exhaust path from the exhaust port.

2.  The temperature control device according to claim 1,
    wherein a width of the exhaust port is narrower than that of the multi-well plate.

3.  The temperature control device according to claim 1,
    wherein a width of the exhaust port is narrowed toward an advancing direction of the air.

4.  The temperature control device according to claim 1,

    wherein a front exhaust port is provided between the air intake port and the multi-well plate, and
    the front exhaust port is narrower in depth than the exhaust port.

5.  The temperature control device according to claim 1,

    wherein the exhaust port is provided with a duct part, and
    an opening of the exhaust port is disposed at a height substantially equal to an upper surface of the multi-well plate.

6.  The temperature control device according to claim 1,
    wherein the exhaust port is provided with a movable lid part.

7.  A biochemical device comprising:
    the temperature control device according to any one of claims 1 to 6.

8.  An analysis device comprising:
    the temperature control device according to any one of claims 1 to 6.

9.  A genetic testing device comprising:
    the temperature control device according to any one of claims 1 to 6.

[FIG.1]

[FIG.2]

[FIG.3]

[FIG.4]

[FIG.5]

[FIG.6]

[FIG.7]

[FIG.8]

[FIG.9]

[FIG.10]

[FIG.11]

[FIG.12]

## INTERNATIONAL SEARCH REPORT

| | International application No. |
|---|---|
| | **PCT/JP2022/030360** |

**A. CLASSIFICATION OF SUBJECT MATTER**

*C12M 1/00*(2006.01)i
FI: C12M1/00 A

According to International Patent Classification (IPC) or to both national classification and IPC

**B. FIELDS SEARCHED**

Minimum documentation searched (classification system followed by classification symbols)

C12M1/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/MEDLINE/EMBASE/BIOSIS (STN), JSTPlus/JMEDPlus/JST7580 (JDreamIII), Japio-GPG/FX, PubMed

**C. DOCUMENTS CONSIDERED TO BE RELEVANT**

| Category* | Citation of document, with indication, where appropriate, of the relevant passages | Relevant to claim No. |
|---|---|---|
| A | JP 3203237 U (THERMOGEN, INC.) 24 February 2016 (2016-02-24)<br>claim 1, fig. 2, 3, paragraph [0034] | 1-9 |
| A | WO 2022/003915 A1 (HITACHI HIGH-TECH CORP.) 06 January 2022 (2022-01-06)<br>claim 2, fig. 2 | 1-9 |
| A | WO 2022/044316 A1 (HITACHI HIGH-TECH CORP.) 03 March 2022 (2022-03-03)<br>claim 1, fig. 6 | 1-9 |

☐ Further documents are listed in the continuation of Box C.    ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **22 September 2022** | **04 October 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2022/030360**

| Patent document cited in search report | | | Publication date (day/month/year) | Patent family member(s) | Publication date (day/month/year) |
|---|---|---|---|---|---|
| JP | 3203237 | U | 24 February 2016 | (Family: none) | |
| WO | 2022/003915 | A1 | 06 January 2022 | (Family: none) | |
| WO | 2022/044316 | A1 | 03 March 2022 | (Family: none) | |

Form PCT/ISA/210 (patent family annex) (January 2015)

**EP 4 570 897 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007097476 A

- JP 2022002505 A